# EUROPEAN PATENT APPLICATION

(11) **EP 4 737 584 A1**
(43) Date of publication of application: **06.05.2026**
(21) Application number: 24832088.9
(22) Date of filing: 28.06.2024
(51) Int. Cl.: C12Q 1/02, C07K 14/525, C12N 5/10, C12N 5/079, C12Q 1/6841, G01N 33/50, G01N 33/53, G01N 33/68

(54) **METHOD FOR EVALUATING INTERCELLULAR INTERACTION OF NEUROINFLAMMATION**

(30) Priority: 30.06.2023 JP 2023108778; 26.04.2024 JP 2024072112
(71) Applicant: FUJIFILM Corporation, Tokyo 106-8620 (JP)
(72) Inventor: KOBAYASHI Hayato, Ashigarakami-gun, Kanagawa 258-8577 (JP); ENDOH Setsu, Ashigarakami-gun, Kanagawa 258-8577 (JP); KATO Hiroshi, Ashigarakami-gun, Kanagawa 258-8577 (JP)
(74) Representative: Hoffmann Eitle
(86) International application number: PCT/JP2024/023480
(87) International publication number: WO 2025/005230

(57) **Abstract**

An object of the present invention is to provide a method of evaluating intercellular interactions in neuroinflammation using a co-culture containing human-derived neural cells capable of mimicking human brain functions. According to the present invention, there is provided a method of evaluating intercellular interactions in neuroinflammation, the method including a step of producing a co-culture containing at least two cells selected from the group consisting of human-derived astrocytes, human-derived neurons, human-derived microglia, and human-derived oligodendrocytes, a step of applying an inflammatory stimulation to a first cell contained in the co-culture, a step of detecting at least one selected from the group consisting of an inflammatory response marker in the cells contained in the co-culture, neural activity of the cells, and cell morphology, and a step of evaluating, over time, a change in at least one selected from the group consisting of the inflammatory response marker, the neural activity, and the cell morphology in the first cell and a second cell different from the first cell contained in the co-culture.

## Description

### Technical Field

The present invention relates to a method of evaluating intercellular interactions in neuroinflammation using a co-culture containing human-derived neural cells.

### Background Art

The central nervous system is composed of neurons and glial cells (astrocytes, microglia, and oligodendrocytes), and these cells exert normal brain functions by influencing each other (Non-Patent Document 1). In addition to normal brain functions such as axonal conduction, synaptic transmission, and information processing (Non-Patent Document 2), it has been reported that intercellular interactions are also important in pathological conditions. For example, it has been reported that microglia are activated by aging or inflammation to affect astrocytes, and the astrocytes further damage neurons, thereby being deeply involved in the pathological mechanisms of neurodegenerative diseases (Non-Patent Documents 3 and 4). Therefore, a method capable of evaluating intercellular interactions between neurons and glial cells is very useful for evaluating the original functions of the human brain and for studying pathological mechanisms.

With respect to intercellular interactions between neurons and glial cells, there is a report in which mutual influences have been evaluated by using co-culture systems of various combinations (Non-Patent Document 5). In this report, it is reported that the amount of cytokine in the culture medium after lipopolysaccharide (LPS) stimulation changes depending on various combinations of cells, including the three-cell-type co-culture of neurons, astrocytes, and microglia, and it can be inferred from this result that different cells influence each other. However, with this method, it is not possible to evaluate which cells are activated when and with which cells they interact. To study the effect of intercellular interactions on normal brain functions or the effect of intercellular interactions on pathological mechanisms, a method capable of evaluating which cells interact when and with which cells is useful.

Patent Document 1 describes results that, in a case where the co-culture system of astrocytes, neurons, and microglia is subjected to LPS stimulation, the astrocytes release C3 in a microglia-dependent manner. Patent Document 2 describes the results of morphological observation by immunostaining and cytokine measurement in the culture medium in a case where the co-culture system of astrocytes, neurons, and microglia is subjected to LPS stimulation.

### Prior Art Documents

### Non-Patent Documents

Non-Patent Document 1: Francesca Cerbai et al., PLoS One, Vol. 7, Article number e45250, 2012
Non-Patent Document 2: R. Douglas Fields et al., Science, Vol. 298, pp. 556-562, 2002
Non-Patent Document 3: Shane A. Liddelow et al., Nature, Vol. 541, pp. 481-487, 2017
Non-Patent Document 4: Laura E. Clarke et al., Proceedings of the National Academy of Sciences of the United States of America, Vol. 115, pp. E1896-E1905, 2018
Non-Patent Document 5: Sudha R. Guttikonda et al., Nature Neuroscience, Vol. 24, pp. 343-354, 2021

### Patent Documents

Patent Document 1: JP2022-511385A
Patent Document 2: WO2023/039567A

### Summary of Invention

### Object to be solved by the invention

An object to be achieved by the present invention is to provide a method of evaluating intercellular interactions in neuroinflammation using a co-culture containing human-derived neural cells, capable of mimicking human brain functions.

### Means for solving the object

As a result of intensive studies to achieve the above object, the present inventors have found that intercellular interactions in neuroinflammation can be evaluated by applying an inflammatory stimulation to first cells contained in a co-culture containing at least two cells selected from the group consisting of astrocytes, neurons, microglia, and oligodendrocytes, and evaluating a change in an inflammatory response marker in the first cells and second cells over time. The present invention has been completed based on these findings.

That is, according to an aspect of the present invention, the following invention is provided.
<1> A method of evaluating intercellular interactions in neuroinflammation, the method comprising:
   a step of producing a co-culture containing at least two cells selected from the group consisting of human-derived astrocytes, human-derived neurons, human-derived microglia, and human-derived oligodendrocytes;
   a step of applying an inflammatory stimulation to first cells contained in the co-culture;
   a step of detecting at least one selected from the group consisting of an inflammatory response marker in the cells contained in the co-culture, neural activity of the cells, and cell morphology; and
   a step of evaluating, over time, a change in at least one selected from the group consisting of the inflammatory response marker, the neural activity, and the cell morphology in the first cells and second cells different from the first cells and contained in the co-culture.
<2> The method according to <1>, in which the astrocytes, the neurons, the microglia, and the oligodendrocytes are induced to differentiate from human-derived pluripotent stem cells.
<3> The method according to <2>, in which the human-derived pluripotent stem cells are human iPS cells.
<4> The method according to any one of <1> to <3>, in which the first cells are human-derived microglia, human-derived astrocytes, or human-derived neurons.
<5> The method according to any one of <1> to <4>, in which, in a case where the first cells are human-derived astrocytes, the second cells are at least one selected from the group consisting of human-derived neurons and human-derived microglia.
<6> The method according to any one of <1> to <4>, in which, in a case where the first cells are human-derived neurons, the second cells are at least one selected from the group consisting of human-derived astrocytes and human-derived microglia.
<7> The method according to any one of <1> to <4>, in which, in a case where the first cells are human-derived microglia, the second cells are at least one selected from the group consisting of human-derived astrocytes and human-derived neurons.
<8> The method according to any one of <1> to <7>, in which the co-culture is a two-dimensional culture or a three-dimensional culture.
<9> The method according to any one of <1> to <8>, in which the inflammatory stimulation is a chemical stimulation.
<10> The method according to <9>, in which the chemical stimulation is a chemical stimulation with at least one substance selected from the group consisting of lipopolysaccharide, rotenone, amyloid-β protein, tau protein, α-synuclein, and TDP-43.
<11> The method according to any one of <1> to <8>, in which the inflammatory stimulation is a stimulation by adding cells different from the cells in the co-culture or adding a culture supernatant obtained by culturing the cells different from the cells in the co-culture.
<12> The method according to any one of <1> to <8>, in which the inflammatory stimulation is induced by aging of the first cells, a genetic mutation in the first cells, or gene introduction into the first cells in the co-culture.
<13> The method according to <12>, in which a protein produced by the genetic mutation in the first cells in the co-culture or the gene introduction into the first cells in the co-culture is at least one selected from the group consisting of amyloid-β protein, tau protein, α-synuclein, and TDP-43.
<14> The method according to any one of <1> to <8>, in which the inflammatory stimulation that stimulates the first cells is induced by a substance secreted from cells different from the first cells in the co-culture.
<15> The method according to any one of <1> to <14>, in which the inflammatory response marker is at least one selected from the group consisting of NF-_{κ}B, TNF-α, and STAT1.
<16> The method according to any one of <1> to <15>, in which the step of detecting the inflammatory response marker in the cells contained in the co-culture is a step of visualizing the inflammatory response marker protein by an immunostaining method.
<17> The method according to any one of <1> to <16>, in which the step of detecting the inflammatory response marker in the cells contained in the co-culture is a step of visualizing an mRNA expression level of the inflammatory response marker gene by a fluorescent in situ hybridization method.
<18> The method according to any one of <1> to <14>, in which the neural activity is at least one selected from the group consisting of intracellular calcium flux and action potential of the cells.
<19> The method according to any one of <1> to <14>, in which the step of detecting the neural activity in the cells contained in the co-culture is a step of visualizing intracellular calcium flux.
<20> The method according to <19>, in which the step of visualizing the intracellular calcium flux is calcium imaging.
<21> The method according to any one of <1> to <14>, in which the step of detecting the neural activity in the cells contained in the co-culture is a step of measuring action potential of the cells.

### Effect of the invention

The method of evaluating intercellular interactions in neuroinflammation according to the present invention can be used for the development of therapeutic agents and exploration of biomarkers for various neurodegenerative diseases associated with neuroinflammation.

### BRIEF DESCRIPTION OF THE DRAWINGS

[Fig.1] FIG. 1 shows a temporal change in localization of NF-κB after LPS stimulation in a co-culture.
[Fig.2] FIG. 2 shows staining of NF-κB and IBA1, which is a marker of microglia, 0.5 hours after LPS stimulation.
[Fig.3] FIG. 3 shows staining of NF-κB and IBA1, which is a marker of microglia, 3 hours after LPS stimulation.
[Fig.4] FIG. 4 shows a temporal change in localization of NF-κB after LPS stimulation in a two-cell-type co-culture of nerve and astrocyte.
[Fig.5] FIG. 5 shows a temporal change in localization of NF-κB after LPS stimulation in a co-culture treated with R-7050.
[Fig.6] FIG. 6 shows staining of NF-κB and IBA1, which is a marker of microglia, 1, 3, and 6 hours after LPS stimulation in a co-culture treated with R-7050.
[Fig.7] FIG. 7 shows a temporal change in localization of STAT1 after LPS stimulation in a three-cell-type co-culture of neurons, astrocytes, and microglia.
[Fig.8] FIG. 8 shows staining of STAT1 and IBA1, which is a marker of microglia, 1 and 6 hours after LPS stimulation in a three-cell-type co-culture of neurons, astrocytes, and microglia.
[Fig.9] FIG. 9 shows a temporal change in localization of STAT1 after LPS stimulation in a two-cell-type co-culture of neurons and astrocytes.
[Fig.10] FIG. 10 shows a temporal change in localization of NF-κB after LPS stimulation in a three-cell-type co-culture of neurons, astrocytes, and microglia containing about 0.11% microglia.
[Fig.11] FIG. 11 shows staining NF-κB and IBA1, which is a marker of microglia, 1 and 3 hours after LPS stimulation in a three-cell-type co-culture of neurons, astrocytes, and microglia containing about 0.11% microglia.
[Fig.12] FIG. 12 shows staining of NF-κB, GFAP, which is a marker of astrocytes, and Neun, which is a marker of neurons, after TNF-α stimulation in a two-cell-type co-culture of neurons and astrocytes.
[Fig.13] FIG. 13 shows an evaluation of BPM (the number of peaks of calcium oscillation per minute) by calcium imaging.

### Embodiments for carrying out the invention

Hereinafter, embodiments of the present invention will be specifically described.

A method of evaluating intercellular interactions in neuroinflammation according to the embodiment of the present invention is a method including a step of producing a co-culture containing at least two cells selected from the group consisting of human-derived astrocytes, human-derived neurons, human-derived microglia, and human-derived oligodendrocytes, a step of applying an inflammatory stimulation to a first cell contained in the co-culture, a step of detecting at least one selected from the group consisting of an inflammatory response marker in the cells contained in the co-culture, neural activity of the cells, and cell morphology, and a step of evaluating, over time, a change in at least one selected from the group consisting of the inflammatory response marker, the neural activity, and the cell morphology in the first cell and a second cell different from the first cell contained in the co-culture.

In the present invention, in a co-culture containing at least two cells selected from the group consisting of astrocytes, neurons, microglia, and oligodendrocytes, after neuroinflammation is induced by a stimulation such as LPS or by introduction of a mutation into the cell or forced expression of a gene, localization or expression level of an inflammatory response marker is evaluated by immunostaining or fluorescent in situ hybridization (FISH) method over time, whereby it is possible to visualize which cells among the plurality of cells are activated at which timing, and to evaluate intercellular interactions by observing a temporal change in the activation.

In addition, in the present invention, in a co-culture containing at least two cells selected from the group consisting of astrocytes, neurons, microglia, and oligodendrocytes, after neuroinflammation is induced by a stimulation such as LPS or by introduction of a mutation into the cell or forced expression of a gene, the neural activity of the cells is evaluated over time using the intracellular calcium flux or the action potential of the cells as an indicator, whereby it is possible to visualize which cells among the plurality of cells are activated at which timing, and to evaluate intercellular interactions by observing a temporal change in the activation.

In addition, in the present invention, in a co-culture containing at least two cells selected from the group consisting of astrocytes, neurons, microglia, and oligodendrocytes, after neuroinflammation is induced by a stimulation such as LPS or by introduction of a mutation into the cell or forced expression of a gene, using morphology of the cells (cell morphology) as an indicator, changes in cell morphology are evaluated over time, whereby it is possible to visualize which cells among the plurality of cells are activated at which timing, and to evaluate intercellular interactions by observing a temporal change in the activation.

The astrocytes, the neurons, the microglia, and the oligodendrocytes are preferably induced to differentiate from human-derived pluripotent stem cells.

Examples of the human-derived pluripotent stem cells include human induced pluripotent stem cells (human iPS cells), human embryonic stem cells (human ES cells), human mesenchymal stem cells, and the like. Human iPS cells are preferable, but the human-derived pluripotent stem cell is not particularly limited thereto. The human iPS cell is an iPS cell produced from a human cell.

Examples of the human-derived pluripotent stem cells include pluripotent stem cells derived from a sample in which there is no mutation in a disease-related gene that causes a neurological disease, pluripotent stem cells derived from a sample collected from a healthy human (healthy person) who does not have a neurological disease, pluripotent stem cells derived from a sample collected from a patient having a disease, and pluripotent stem cells derived from a sample in which there is a mutation in a disease-related gene. The "there is no mutation in a disease-related gene" means that there is no mutation that causes a nervous system disease in the disease-related gene. That is, in a case where there is a mutation in a gene but the mutation does not cause a disease, it is interpreted that there is no mutation in the disease-related gene. In addition, in a case of "there is a mutation in a disease-related gene", the mutation may be an endogenous genetic mutation originally possessed by a patient, or an exogenous genetic mutation introduced artificially. It is presumed that, by producing the co-culture according to the embodiment of the present invention using the pluripotent stem cells derived from a sample collected from a patient having a disease or the pluripotent stem cells derived from a sample in which there is a mutation in a disease-related gene, the pathological conditions of a disease derived from a sample or a disease caused by a mutation in a disease-related gene can be mimicked.

The ES cells can be established, for example, by culturing an early embryo before implantation, an inner cell mass constituting the early embryo, a single-cell embryo, or the like (Manipulating the Mouse Embryo, A Laboratory Manual, Second Edition, Cold Spring Harbor Laboratory Press, 1994, Thompson, J. A. et al.: Science, 282, 1145-1147, 1998). As the early embryo, an early embryo produced by nuclear transplantation of a somatic cell nucleus may be used (Wilmut et al.: Nature, 385, 810, 1997; Cibelli et al.: Science, 280, 1256, 1998; Akira Iritani et al.: Protein, Nucleic Acid and Enzyme, 44, 892, 1999; Baguisi et al.: Nature Biotechnology, 17, 456, 1999; Wakayama et al.: Nature, 394, 369, 1998, Nature Genetics, 22, 127, 1999, Proc. Natl. Acad. Sci. USA, 96, 14984, 1999; Rideout III et al.: Nature Genetics, 24, 109, 2000; Tachibana et al.: Human Embryonic Stem Cells Derived by Somatic Cell Nuclear Transfer, Cell, 2013, in press). As the early embryo, a parthenogenetic embryo may be used (Kim et al.: Science, 315, 482-486, 2007; Nakajima et al.: Stem Cells, 25, 983-985, 2007; Kim et al.: Cell Stem Cell, 1, 346-352, 2007; Revazova et al.: Cloning Stem Cells, 9, 432-449, 2007; Revazova et al.: Cloning Stem Cells, 10, 11-24, 2008). In addition to the above-described papers, for the production of the ES cells, Strelchenko N, et al.: Reprod Biomed Online. 9:623-629, 2004, Klimanskaya I, et al.: Nature 444:481-485, 2006, Chung Y, et al.: Cell Stem Cell 2:113-117, 2008, Zhang X, et al.: Stem Cells 24:2669-2676, 2006, Wassarman, P. M. et al.: Methods in Enzymology, Vol. 365, 2003, and the like are useful as references. It is noted that a fused ES cell obtained by cell fusion of an ES cell with a somatic cell is also included in the embryonic stem cell that is used in the method according to the embodiment of the present invention.

Some ES cells are available from conservation institutions or are commercially available. For example, human ES cells are available from the Institute for Frontier Medical Sciences, Kyoto University (for example, KhES-1, KhES-2, and KhES-3), WiCell Research Institute, ESI BIO.

The iPS cell means a cell that is produced by reprogramming a somatic cell by introducing reprogramming factors and has pluripotency (multiple differentiation potency) and proliferation ability. The iPS cells exhibit properties similar to the ES cells. The somatic cells used for producing iPS cells are not particularly limited and may be differentiated somatic cells or undifferentiated stem cells. The iPS cell can be prepared by a known method or the like. In addition, it is naturally expected that an iPS cell production method to be developed in the future will be applied.

The most basic method for producing the iPS cells is a method of introducing four factors of Oct3/4, Sox2, Klf4, and c-Myc, which are transcription factors, into cells using a virus (Takahashi K, Yamanaka S: Cell 126 (4), 663-676, 2006; Takahashi K, et al.: Cell 131 (5), 861-72, 2007). For the human iPS cells, there is a report of establishment by introduction of four factors of Oct4, Sox2, Lin28, and Nanog (Yu J, et al.: Science 318 (5858), 1917-1920, 2007). The establishment of iPS cells by introduction of three factors excluding c-Myc (Nakagawa M, et al.: Nat. Biotechnol. 26 (1), 101-106, 2008), two factors of Oct3/4 and Klf4 (Kim J B, et al.: Nature 454 (7204), 646-650, 2008), or only Oct3/4 (Kim J B, et al.: Cell 136 (3), 411-419, 2009) has also been reported. In addition, a method of introducing a protein, which is an expression product of a gene, into a cell has also been reported (Zhou H, Wu S, Joo JY, et al.: Cell Stem Cell 4, 381-384, 2009; Kim D, Kim CH, Moon JI, et al.: Cell Stem Cell 4, 472-476, 2009). On the other hand, it has also been reported that the production efficiency can be improved or the number of factors to be introduced can be reduced by using an inhibitor BIX-01294 for histone methyltransferase G9a, a histone deacetylase inhibitor valproic acid (VPA), BayK8644, or the like (Huangfu D, et al.: Nat. Biotechnol. 26 (7), 795-797, 2008; Huangfu D, et al.: Nat. Biotechnol. 26 (11), 1269-1275, 2008; Silva J, et al.: PLoS. Biol. 6 (10), e253, 2008). Studies on the gene introduction method are also being advanced, and in addition to the retrovirus, a technology has been developed in which a lentivirus (Yu J, et al.: Science 318 (5858), 1917-1920, 2007), an adenovirus (Stadtfeld M, et al.: Science 322 (5903), 945-949, 2008), a plasmid (Okita K, et al.: Science 322 (5903), 949-953, 2008), a transposon vector (Woltjen K, Michael IP, Mohseni P, et al.: Nature 458, 766-770, 2009; Kaji K, Norrby K, Pac a A, et al.: Nature 458, 771-775, 2009; Yusa K, Rad R, Takeda J, et al.: Nat Methods 6, 363-369, 2009), or an episomal vector (Yu J, Hu K, Smuga-Otto K, Tian S, et al.: Science 324, 797-801, 2009) is used for gene introduction.

Cells transformed to iPS cells, that is, cells that have undergone initialization (reprogramming) can be selected using, as an index, the expression of pluripotent stem cell markers (undifferentiated markers) such as Fbxo15, Nanog, Oct3/4, Fgf-4, Esg-1, and Cript, or the like. The selected cells can be recovered as iPS cells.

As a method of producing the iPS cells, in addition to a method of manufacturing the iPS cells by direct initialization using gene expression, the iPS cells can also be induced from the somatic cells by adding a compound (Hou P, et al.: Science 341 (6146), 651-654, 2013).

The iPS cells can be provided from, for example, FUJIFILM Cellular Dynamics, Inc. (FCDI), National University Corporation Kyoto University, or Independent Administrative Institution RIKEN BioResource Research Center.

Examples of the human-derived astrocytes include astrocytes induced to differentiate from astrocyte precursor cells (XCell Science, XCS-AP-001-1V, and the like), iCell (registered trademark) astrocytes (FUJIFILM Cellular Dynamics, C1037), and the like, and astrocytes induced to differentiate from astrocyte precursor cells are preferable. In a case of inducing the astrocyte precursor cells to differentiate into the astrocytes, it is preferable to induce the astrocyte precursor cells to differentiate in the absence of serum.

Examples of the method for obtaining human-derived neurons include inducing from human iPS cells produced from somatic cells collected from healthy humans (healthy person) who do not have a mutation of a disease-related gene causing a nervous system disease or who do not have a nervous system disease, or from somatic cells collected from patients who have a nervous system disease, and inducing from an established human iPS cell.

The neurons induced to differentiate from the human-derived pluripotent stem cells are not particularly limited, but are preferably motor neurons, cerebral cortex excitatory neurons, or substantia nigra neurons.

A method for inducing human-derived pluripotent stem cells to differentiate into neurons is not particularly limited, but includes a method of producing neural stem cells from pluripotent stem cells using a low-molecular-weight compound treatment or the like and then inducing to differentiate into neurons, and a method of directly inducing to differentiate into neurons by gene expression or the like.

Examples of the method of inducing the pluripotent stem cells to differentiate into the neurons include:
(1) a method of forming and differentiating embryoid bodies (cell aggregates containing neural precursor cells) by culturing in a serum-free medium (SFEB method: Watanabe K, et al., Nat. Neurosci., 8:288-296, 2005; SFEBq method: Wataya T, et al. Proc. Natl. Acad. Sci. USA., 105:11796-11801, 2008);
(2) a method of differentiating by culturing on stromal cells (SDIA method: Kawasaki H, et al., Neuron, 28:31-40, 2000);
(3) a method of differentiating by culturing on Matrigel to which a drug has been added (Chambers S. M, et al., Nat. Biotechnol., 27:275-280, 2009);
(4) a method of differentiating by culturing in a culture medium containing a low-molecular-weight compound as a substitute for a cytokine (US5843780A),
(5) a method of differentiating by introducing a neurogenic factor (a gene encoding Neurogenin2 (NGN2) protein or the like) into the pluripotent stem cells and expressing the factor (WO2014/148646A; and Zhang Y, et al., Neuron, 78:785-98, 2013); and
(6) a method of differentiating by introducing and expressing miR-9/9*-124 in pluripotent stem cells; and
a combination of these methods.

Among the above, (5) the method of introducing a gene encoding an NGN2 protein into the pluripotent stem cells and expressing the protein is preferable because matured neurons can be obtained in a short period of time and with high efficiency.

Examples of the human-derived neurons include induced neurons differentiated from iPS cells by forced expression of NGN2, iCell (registered trademark) GlutaNerve (FUJIFILM Cellular Dynamics, C1033), iCell (registered trademark) GABANerve (FUJIFILM Cellular Dynamics, C1008), iCell (registered trademark) DopaNerve (FUJIFILM Cellular Dynamics, C1028), iCell (registered trademark) Motor Nerve (FUJIFILM Cellular Dynamics, C1048), and the like. Among these, the induced neurons differentiated from iPS cells by forced expression of NGN2 and the iCell (registered trademark) GlutaNerve are preferable.

Examples of the human-derived microglia include iCell (registered trademark) Microglia (FUJIFILM Cellular Dynamics, C1110), microglia (Axol Bioscience, AX0664), and the like. Among these, the iCell (registered trademark) Microglia is preferable.

Examples of the human-derived oligodendrocytes include induced oligodendrocytes differentiated from iPS cells by forced expression of SRY-box transcription factor 10 (SOX10), Oligodendrocyte lineage transcription factor 2 (OLIG2), and NK6 homeobox 2 (NKX6-2), induced oligodendrocytes differentiated from iPS cells by forced expression of SOX10 and OLIG2, induced oligodendrocytes differentiated from iPS cells by forced expression of SOX10, oligodendrocytes induced to differentiate from iPS cells by adding a substance that promotes differentiation into oligodendrocytes to a culture medium (Panagiotis Douvaras, et al., Nat Protocols., 10:1143-54, 2015; Tomoko Yamashita, et al., PLoS One., 12:e0171847.), and the like. Among these, the induced oligodendrocytes differentiated from iPS cells by forced expression of SOX10, OLIG2, and NKX6-2 are preferable.

The astrocytes are cells that express at least one or more marker genes specific to the astrocytes consisting of glial fibrillary acidic protein (GFAP), S100 calcium binding protein B (S100β), potassium inwardly rectifying channel subfamily J member 10 (KCNJ10), Aquaporin-4 (AQP4), and solute carrier family 1 member 3 (SLC1A3).

The neurons are preferably cells which express at least one or more marker genes specific to neurons consisting of β-III tubulin, NeuN, a neural cell adhesion molecule (N-CAM), and microtubule-associated protein 2 (MAP2), and have a β-III tubulin-positive protrusion (hereinafter, referred to as a neurite).

Microglia are cells that express at least one or more marker genes specific to microglia consisting of ionized calcium-binding adapter molecule 1 (IBA1), CD33, CD45, triggering receptor expressed on myeloid cells 2 (TREM2), purinergic receptor P2Y (P2RY12, G-protein coupled 12), transmembrane protein 119 (TMEM119), and CX3C-chemokine receptor 1 (CX3CR1).

The oligodendrocytes are cells that express at least one or more marker gene specific to the oligodendrocytes consisting of OLIG2, oligodendrocyte marker O4 (O4), and myelin basic protein (MBP).

The expression level of a marker gene can be usually analyzed by the production amount of a transcript corresponding to the gene, or the production amount, the activity, and the like of a translation product thereof. The measurement of the expression level can be carried out by measuring an mRNA which is a transcript of a gene or a protein which is a translation product of a gene; however, it is preferably carried out by measuring an mRNA or a cDNA which is a reverse transcript thereof. The detection or measurement of the expression of the translation product (protein) can be performed by immunocytochemistry for detecting the protein in the cell using an antibody.

**In** the present invention, a co-culture containing at least two cells selected from the group consisting of human-derived astrocytes, human-derived neurons, human-derived microglia, and human-derived oligodendrocytes is produced. Examples of the combination of cells to be used include a combination of astrocytes and neurons, a combination of astrocytes and microglia, a combination of astrocytes and oligodendrocytes, a combination of neurons and microglia, a combination of neurons and oligodendrocytes, a combination of microglia and oligodendrocytes, a combination of astrocytes, neurons, and microglia, a combination of astrocytes, neurons, and oligodendrocytes, a combination of astrocyte, microglia, and oligodendrocytes, a combination of neurons, microglia, and oligodendrocytes, and a combination of astrocytes, neurons, microglia, and oligodendrocytes, but the combination is not particularly limited.

For example, in a case of using astrocytes, neurons, and microglia, a proportion of the astrocytes among all cells used is 30% or more, preferably 35% or more, more preferably 40% or more, and still more preferably 45% or more. A lower limit of the proportion of the astrocytes among all the cells used is not particularly limited, as long as one or more cells are present in the culture vessel, and is generally 0.1% or more, preferably 1% or more, more preferably 5% or more, and still more preferably 10% or more. An upper limit of the proportion of the astrocytes among all the cells used is not particularly limited, but is generally 80% or less and preferably 70% or less. A proportion of the microglia among all cells used is preferably 50% or less, more preferably 40% or less, and still more preferably 30% or less. A lower limit of the proportion of the microglia among all the cells used is not particularly limited, as long as one or more cells are present in the culture vessel, and is generally 0.1% or more, preferably 1% or more, more preferably 2% or more, still more preferably 5% or more, and particularly preferably 10% or more. An upper limit of the proportion of the microglia among all the cells used is not particularly limited, but is generally 65% or less and preferably 60% or less. A proportion of the neurons among all the cells used is preferably 10% or more, more preferably 15% or more, still more preferably 20% or more, and particularly preferably 25% or more. A lower limit of the proportion of the neurons among all the cells used is not particularly limited, as long as one or more cells are present in the culture vessel, and is generally 0.1% or more, preferably 1% or more, and more preferably 5% or more. An upper limit of the proportion of the neurons among all the cells used is not particularly limited, but is generally 65% or less and preferably 60% or less.

In the present invention, the above-described cells are co-cultured in a culture container.

As the culture container, a plate having wells, a dish, a cell culture insert, a cell culture flask, or the like can be used, and the plate having wells is preferable. Specific examples thereof include ViewPlate (PerkinElmer), a 96-well microplate (Greiner), a 96-well polystyrene microplate (Corning), a PrimeSurface (registered trademark) plate (Sumitomo Bakelite), a cell-repellent plate (Greiner Bio one), and the like, and ViewPlate (PerkinElmer) and a PrimeSurface (registered trademark) plate (Sumitomo Bakelite) are preferable.

Examples of the shape of the culture container include a flat bottom, a round bottom, a U-shaped bottom, a V-shaped bottom, and the like, but the shape is not particularly limited.

In a case where the co-culture is a three-dimensional culture, the properties of the surface with which the culture medium in the container comes into contact are preferably cell non-adhesiveness. As a result, the cells are cultured in a floating state, and a spheroid is easily formed. Alternatively, only a certain portion of the surface inside the container may be cell adhesiveness, and the other portions may be cell non-adhesiveness. In this case, cells can be gathered at the portion with cell adhesiveness to form a spheroid.

In a case where the co-culture is a two-dimensional culture, the lower limit value of the cell density (the cell density is the total cell density of two or more cells to be used, the same applies to the following) at the time of seeding into the culture container is not particularly limited, but it is, for example, preferably 2.5 × 10⁴ cells/cm² or more, more preferably 5.0 × 10⁴ cells/cm² or more, still more preferably 10.0 × 10⁴ cells/cm² or more, even still more preferably 15.0 × 10⁴ cells/cm² or more, particularly preferably 20.0 × 10⁴ cells/cm² or more, and most preferably 25.0 × 10⁴ cells/cm² or more. The upper limit value of the cell density at the time of seeding in the culture container is not particularly limited, but for example, may be 60.0 × 10⁴ cells/cm² or less, and is preferably less than 55.0 × 10⁴ cells/cm², more preferably 50.0 × 10⁴ cells/cm² or less, still more preferably 45.0 × 10⁴ cells/cm² or less, even still more preferably 40.0 × 10⁴ cells/cm² or less, and particularly preferably 35.0 × 10⁴ cells/cm² or less.

In a case where the co-culture is a two-dimensional culture, the cell density at the time of seeding in the culture container is preferably 5.0 × 10⁴ cells/cm² or more and 55.0 × 10⁴ cells/cm² or less, more preferably 10.0 × 10⁴ cells/cm² or more and 50.0 × 10⁴ cells/cm² or less, still more preferably 15.0 × 10⁴ cells/cm² or more and 45.0 × 10⁴ cells/cm² or less, even still more preferably 20.0 × 10⁴ cells/cm² or more and 40.0 × 10⁴ cells/cm² or less, and particularly preferably 25.0 × 10⁴ cells/cm² or more and 35.0 × 10⁴ cells/cm² or less.

In a case where the co-culture is a three-dimensional culture, the lower limit value of the number of cells (the number of cells is the total number of cells of two or three types of cells, the same applies to the following) at the time of seeding into the 96-well plate is not particularly limited, but it is, for example, preferably 0.7 × 10⁴ cells/well or more, more preferably 1.0 × 10⁴ cells/well or more, still more preferably 1.2 × 10⁴ cells/well or more, even still more preferably 1.4 × 10⁴ cells/well or more, particularly preferably 1.6 × 10⁴ cells/well or more, and most preferably 1.8 × 10⁴ cells/well or more. The upper limit value of the cell density at the time of seeding in the culture container is not particularly limited, but for example, may be 8.0 × 10⁴ cells/well or less, and is preferably less than 8.0 × 10⁴ cells/well, more preferably 5.0 × 10⁴ cells/well or less, still more preferably 3.0 × 10⁴ cells/well or less, even still more preferably 2.5 × 10⁴ cells/well or less, and particularly preferably 2.3 × 10⁴ cells/well or less.

In a case where the co-culture is a three-dimensional culture, the number of cells at the time of seeding into the 96-well plate is preferably 1.0 × 10⁴ cells/well or more and 8.0 × 10⁴ cells/well or less, more preferably 1.2 × 10⁴ cells/well or more and 5.0 × 10⁴ cells/well or less, still more preferably 1.4 × 10⁴ cells/well or more and 3.0 × 10⁴ cells/well or less, even still more preferably 1.6 × 10⁴ cells/well or more and 2.5 × 10⁴ cells/well or less, and particularly preferably 1.8 × 10⁴ cells/well or more and 2.3 × 10⁴ cells/well or less.

A medium can be selected from the known media and commercially available media. The culture medium used for the culture can be used by adding an additive to the basal medium. Here, examples of the basal medium include DMEM, DMEM (Dulbecco's modified Eagle's medium)/F12, BrainPhys (registered trademark) Neuronal Medium, Neurobasal (trademark) Medium-A, Neurobasal (trademark) Medium, Neural Progenitor Basal Medium, NS-A Basal Medium, Basal Medium Eagle (BME), BGJb Medium, CMRL 1066 Medium, Glasgow Minimum Essential Medium (MEM), Improved MEM Zinc Option, Iscove's Modified Dulbecco's Medium (IMDM), Medium 199, Eagle MEM, αMEM, Ham's F12 Medium, RPMI 1640 Medium, Fischer's Medium, and the like. In addition, as the culture medium, a single culture medium may be used, or two or more culture media may be used in combination.

Specific examples of the additive that can be added to the culture medium include serum, retinoic acid, Wnt, bone morphogenetic protein (BMP) (BMP-4 and the like), ciliary neurotrophic factor (CNTF), brain-derived neurotrophic factor (BDNF), glial cell line-derived neurotrophic factor (GDNF), basic fibroblast growth factor (bFGF), epidermal growth factor (EGF), hepatocyte growth factor (HGF), sonic hedgehog (Shh), insulin-like growth factor 1 (IGF-1), Activin A, Heregulinβ-1, interleukins, 8-Br-cAMP, heparin, heparan sulfate, laminin, collagen, fibronectin, progesterone, selenite, B-27 (registered trademark) supplement, N2 supplement with transferrin (Apo), N2 supplement with transferrin, GlutaMAX (trademark), L(+)-ascorbic acid, ITS supplement, MEM non-essential amino acids, and the like, but the additive is not particularly limited. Furthermore, an antibiotic (penicillin, streptomycin, or the like) may be added.

As the culture conditions, general cell culture conditions may be selected. For example, conditions of 37°C and 5% CO₂.are mentioned. The culture medium may be exchanged at appropriate intervals (preferably once every 1 to 7 days and more preferably once every 2 to 3 days) during the culture, but the culture medium may not be exchanged during the culture period.

The co-culture may be any of a two-dimensional culture or a three-dimensional culture. The three-dimensional culture may have a spheroid shape.

In the present invention, a step of applying an inflammatory stimulation to the first cells contained in the co-culture is performed.

The first cells are preferably human-derived microglia, human-derived astrocytes, or human-derived neurons. The first cells may be one type or two or more types, but is not particularly limited, and for example, one type is preferable.

In the present invention, even in a case where the inflammatory stimulation to the first cells induces a change in the inflammatory response marker of the second cells different from the first cells, in a case where the change in the inflammatory response marker of the second cells induced by the first cells to which the inflammatory stimulation is applied can be distinguished, the second cells different from the first cells are not regarded as being stimulated by the inflammatory stimulation to the first cells.

As an example of the inflammatory stimulation, a chemical stimulation can be mentioned. Examples of the chemical stimulation include a chemical stimulation with at least one substance selected from the group consisting of lipopolysaccharide, rotenone, amyloid-β protein, tau protein, α-synuclein, and TAR DNA-binding protein 43 (TDP-43), but the chemical stimulation is not particularly limited.

As another example of the inflammatory stimulation, a stimulation by adding cells different from cells in the co-culture or adding a culture supernatant obtained by culturing cells different from cells in the co-culture can be mentioned.

Further other examples of the inflammatory stimulation include an inflammatory stimulation induced by aging, a genetic mutation, or gene introduction of the first cells in the co-culture. For example, in a case where the first cells in the co-culture are astrocytes, aged astrocytes can be used as the inflammatory stimulation. As the inflammatory stimulation induced by a genetic mutation or gene introduction, genetically engineered cells may be used as the inflammatory stimulation.

Examples of the protein produced by a genetic mutation of the first cells in the co-culture or gene introduction into the first cells in the co-culture include at least one selected from the group consisting of amyloid-β protein, tau protein, α-synuclein, and TDP-43, but are not particularly limited.

The inflammatory stimulation that stimulates the first cells may be induced by a substance secreted from cells different from the first cells in the co-culture.

As further another example of the inflammatory stimulation, a physical stimulation can be mentioned. Examples of the physical stimulation include ultraviolet (UV) irradiation, a mechanical stimulation (for example, a pipetting procedure with a culture pipette), but are not particularly limited.

The step of applying an inflammatory stimulation to the first cells contained in the co-culture can be performed, for example, for 10 minutes or more, preferably 20 minutes or more, more preferably 30 minutes or more, still more preferably 1 hour or more, even still more preferably 2 hours or more, and yet even still more preferably 3 hours or more. The step of applying an inflammatory stimulation to the first cells contained in the co-culture can be performed, for example, for 120 hours or less, preferably 96 hours or less, more preferably 72 hours or less, and still more preferably 48 hours or less.

In the present invention, a step of detecting at least one selected from the group consisting of an inflammatory response marker in the cells contained in the co-culture, neural activity of the cells, and cell morphology, and a step of evaluating, over time, a change in at least one selected from the group consisting of the inflammatory response marker, the neural activity, and the cell morphology in the first cell and a second cell different from the first cell contained in the co-culture are performed.

In the present invention, in the evaluation of the intercellular interaction between the first cells and the second cells different from the first cells, it is also possible to evaluate whether or not the function of the first cells is affected by the activation or suppression of the second cells after the second cells are activated or suppressed as a result of giving the inflammatory stimulation to the first cells. In this case, it is assumed that a step of evaluating, over time, a change in at least one selected from the group consisting of the inflammatory response marker, the neural activity, and the cell morphology in the first cell is performed.

As a first example, in a case where the first cells are human-derived astrocytes, the second cells are preferably at least one selected from the group consisting of human-derived neurons and human-derived microglia.

As a second example, in a case where the first cells are human-derived neurons, the second cells are preferably at least one selected from the group consisting of human-derived astrocytes and human-derived microglia.

As a third example, in a case where the first cells are human-derived microglia, the second cells are preferably at least one selected from the group consisting of human-derived astrocytes and human-derived neurons.

In the present invention, in addition to the evaluation of the intercellular interaction in the first cell and the second cell, a step of evaluating, over time, a change in at least one selected from the group consisting of the inflammatory response marker, the neural activity, and the cell morphology in third cells different from the first cells and the second cells may be performed. In this case, it is possible to evaluate whether or not the function of the third cells different from the first cells and the second cells are affected by the second cells after the second cells different from the first cells are activated or suppressed as a result of giving the inflammatory stimulation to the first cells.

In a case of evaluating three different types of intercellular interactions, for example, as the first example, in a case where the first cells are human-derived astrocytes, the second cells are human-derived neurons, and the third cells are human-derived microglia. In addition, as another aspect, in a case where the first cells are human-derived astrocytes, the second cells are human-derived microglia, and the third cells are human-derived neurons.

As a second example, in a case where the first cells are human-derived neurons, the second cells are human-derived astrocytes and the third cells are a human-derived microglia. In addition, as another aspect, in a case where the first cells are human-derived neurons, the second cells are human-derived microglia and the third cells are human-derived astrocytes.

As a third example, in a case where the first cells are human-derived microglia, the second cells are human-derived astrocytes and the thirds cells are human-derived neurons. In addition, as another aspect, in a case where the first cells are human-derived microglia, the second cells are human-derived neurons and the third cells are human-derived astrocytes.

In a case of evaluating three different types of intercellular interactions, in a case where the first cells are human-derived microglia, the second cells are preferably human-derived astrocytes and the third cells are human-derived neurons.

In the present invention, the intercellular interaction can be evaluated by observing the inflammatory response marker over time after stimulating the first cells in the co-culture.

The inflammatory response marker is preferably at least one selected from the group consisting of NF-κB, TNF-α, and STAT1, more preferably NF-κB or STAT1, and particularly preferably NF-κB.

In the present invention, from the viewpoint of evaluating whether or not the intercellular interaction occurs between the first cells and the second cells, the inflammatory response marker of the second cells is an inflammatory response marker induced by the first cells to which the inflammatory stimulation is applied.

In the present invention, from the viewpoint of evaluating whether or not the intercellular interaction occurs between the second cells and the third cells, the inflammatory response marker of the third cells is an inflammatory response marker induced by the second cells that has interacted with the first cells.

In the present invention, various markers such as NF-κB, STAT1, or mRNA of TNF-α can be selected as the inflammatory response marker, and a wide range of activations and intercellular interactions accompanying the activations can be evaluated. **In** addition, the intercellular interaction can be evaluated by capturing the change in the initial stage of the reaction for gene expression or a change in localization of a protein.

Specifically, it is preferable to observe a change in localization of NF-κB, a change in localization of STAT1, a change in mRNA expression level of TNF-α, and the like.

The step of detecting the inflammatory response marker in the cells contained in the co-culture may be, for example, a step of visualizing an inflammatory response marker protein by an immunostaining method, or a step of visualizing an mRNA expression level of the inflammatory response marker gene by a fluorescent in situ hybridization (FISH) method.

The step of evaluating the change in the inflammatory response marker over time is preferably performed by continuously measuring the change in the visualized inflammatory response marker for, for example, 0 hours to 7 days, preferably 0 hours to 3 days, more preferably 0 hours to 48 hours, and still more preferably 0 hours to 24 hours. The intercellular interaction can be evaluated by visualizing, by the immunostaining method or the FISH method, in which cells and when the inflammatory response marker has changed.

In the present invention, the intercellular interaction can be evaluated by observing a change in neural activity over time after stimulating the first cells in the co-culture.

The neural activity is preferably at least one selected from the group consisting of an intracellular calcium flux and an action potential of the cell, and more preferably an intracellular calcium flux.

In the present invention, from the viewpoint of evaluating whether or not the intercellular interaction occurs between the first cells and the second cells, the neural activity of the second cells is a neural activity induced by the first cells to which the inflammatory stimulation is applied.

In the present invention, from the viewpoint of evaluating whether or not the intercellular interaction occurs between the second cells and the third cells, the neural activity of the third cells is a neural activity induced by the second cells that has interacted with the first cells.

In the present invention, various indicators such as an intracellular calcium flux or an action potential of the cell can be selected as the neural activity, and a wide range of activations and intercellular interactions accompanying the activations can be evaluated.

Specifically, it is preferable to observe a change in the intracellular calcium flux or a change in the action potential of the cell.

The step of detecting the neural activity in the cells contained in the co-culture may be, for example, a step of visualizing the intracellular calcium flux. Examples of the method of visualizing the intracellular calcium flux include calcium imaging. In addition, by visualizing the intracellular calcium flux by calcium imaging and measuring calcium oscillation, for example, the number of peaks of calcium oscillation per minute (beats per minute, BPM) can be evaluated.

In addition, the step of detecting the neural activity in the cells contained in the co-culture mayt be, for example, a step of measuring the action potential of the cells. Examples of the step of measuring the action potential of the cell include measuring a change in the action potential of each cell using a microelectrode array (MEA) or measuring a change in the number of network bursts or the peak interval between the cells.

The step of evaluating the change in neural activity over time is preferably performed by continuously measuring the change in the detected neural activity for, for example, 0 hours to 7 days, preferably 0 hours to 3 days, more preferably 0 hours to 48 hours, and still more preferably 0 hours to 24 hours. The intercellular interaction can be evaluated by visualizing, by the MEA method, in which cells and when the neural activity has changed.

In the present invention, the intercellular interaction can be evaluated by observing a change in cell morphology over time after stimulating the first cells in the co-culture.

In the present invention, from the viewpoint of evaluating whether or not the intercellular interaction occurs between the first cells and the second cells, the change in morphology of the second cells is a change in cell morphology induced by the first cells to which the inflammatory stimulation is applied.

In the present invention, from the viewpoint of evaluating whether or not the intercellular interaction occurs between the second cells and the third cells, the change in morphology of the third cells is a change in cell morphology induced by the second cells that has interacted with the first cells.

The step of detecting the cell morphology in the co-culture may include, for example, a step of visualizing the cells contained in the co-culture by immunostaining with a marker protein specific to each cell. Furthermore, examples thereof include a step of visualizing the cells sampled over time by immunostaining to capture an image of the cells and measuring the change in cell morphology by analyzing the obtained images.

The step of evaluating the change in cell morphology over time is preferably performed by continuously evaluating the change in the detected cell morphology for, for example, 0 hours to 7 days, preferably 0 hours to 3 days, more preferably 0 hours to 48 hours, and still more preferably 0 hours to 24 hours. The intercellular interaction can be evaluated by visualizing, by analyzing the temporal change in the obtained image, in which cells and when the cell morphology has changed.

The present invention will be more specifically described with reference to Examples, but the present invention is not limited to the scope of Examples.

### Examples

### Test Example 1: Evaluation of intercellular interaction by immunostaining

### <1> Production of two-dimensional co-culture of astrocytes, neurons, and microglia

Matrigel basement membrane matrix (Corning, 356234) diluted 120-fold with DMEM/F12 (Life technologies, 11320-033) was added to a 96-well plate at 65 µL/well and allowed to stand at 4°C. After 24 hours, the culture medium was replaced with a progenitor cell culture medium and used.

Astrocyte precursor cells (XCell Science, XCS-AP-001-1V) were seeded in a 96-well plate at a density of 5 × 10⁴ cells/well and cultured under the conditions of 37°C and 5% CO₂. The next day, the culture medium was replaced with the differentiation-inducing culture medium and further cultured for 5 days to differentiate into astrocytes.

The neurons were produced by forcing expression of the Ngn2 gene from iPS cells (WO2014/148646A; and Zhang Y, et al., Neuron, 78:785-98, 2013), and the cells that had been frozen and stored were used. The cells that had been frozen and stored were thawed in a warm bath at 37°C, and after thawing, the cells were added to the co-culture medium and centrifuged at 600 × g at room temperature for 5 minutes. After centrifugation, the supernatant was removed, the cells were suspended in 1 mL of the co-culture medium, and the number of cells was counted.

As the microglia, iPS cell-derived microglia (iCell (registered trademark) Microglia, FCDI, C1110) were used. The cells that had been frozen and stored were thawed in a warm bath at 37°C, and after thawing, the cells were added to the co-culture medium and centrifuged at 600 × g at room temperature for 5 minutes. After centrifugation, the supernatant was removed, the cells were suspended in 1 mL of the co-culture medium, and the number of cells was counted.

The neurons and the microglia were mixed in a ratio of 3:1 and seeded on the astrocytes at 4 × 10⁴ cells/well. The cells were cultured under the conditions of 37°C and 5% CO₂, and half of the culture medium was replaced three times a week.

### <2> Activation of microglia and cell fixation

Microglia were specifically stimulated by adding LPS (FUJIFILM Wako Pure Chemical Corporation, 124-05151) at 100 ng/mL to the two-dimensional co-culture that had been cultured for 4 weeks. The cells were fixed by treating, for 30 minutes, with a formaldehyde solution (FUJIFILM Wako Pure Chemical Corporation, 061-00416) diluted 10-fold with PBS(-) (FUJIFILM Wako Pure Chemical Corporation, 166-23555) without treatment, and at 0.5 hours, 1 hour, 3 hours, 6 hours, and 24 hours after the treatment.

In addition, in the two-dimensional co-culture that had been cultured for 19 days, LPS (FUJIFILM Wako Pure Chemical Corporation, 124-05151) was added at 100 ng/mL and R-7050 (Cayman Chemical Company, 16870), which is a TNFR1 inhibitor, was added at 10 µmol/L, and the effect of TNF-α on the intercellular interaction induced by LPS stimulation was evaluated. The cells were fixed by treating, for 30 minutes, with a formaldehyde solution (FUJIFILM Wako Pure Chemical Corporation, 061-00416) diluted 10-fold with PBS(-) (FUJIFILM Wako Pure Chemical Corporation, 166-23555) without treatment, and at 0.5 hours, 1 hour, 3 hours, 6 hours, and 24 hours after the treatment.

### <3> Localization evaluation of NF-κB

The fixed cells were washed with PBS(-), and blocking and permeabilization were performed by treating with a solution (1% BSA/0.2% Triton X-100) in which BSA (Sigma-Aldrich, A4161) and Triton (registered trademark) X-100 (BioVision, 2104-100) were diluted to 1% and 0.2% with PBS(-) for 30 minutes. Thereafter, as a primary antibody reaction, the cells were treated with an anti-IBA1 antibody (FUJIFILM Wako Pure Chemical Corporation, 011-27991) diluted 800-fold with 1% BSA/0.2% Triton X-100 and an anti-NF-κB antibody (Cell Signaling, 8242) diluted 400-fold, and allowed to stand at 4°C overnight.

The next day, the cells were washed with PBS(-), then as a secondary antibody reaction, treated with Donkey anti-goat Alexa Fluor (registered trademark) 488 (Thermo Fisher Scientific, A11055) diluted 1000-fold, Donkey anti-rabbit Alexa Fluor (registered trademark) 594 (Thermo Fisher Scientific, A32754) diluted 1000-fold, and Hoechst (registered trademark) 33342 (Dojindo, 346-07951) diluted 1000-fold as a secondary antibody reaction, and allowed to stand at room temperature for 60 minutes.

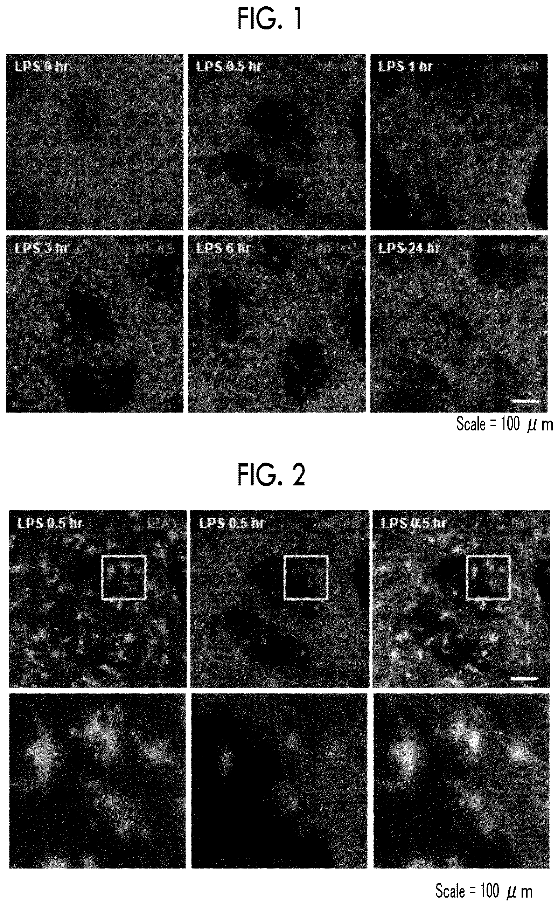

After washing with PBS(-), the cells were imaged with a fluorescence microscope (Nikon, ECLIPSE Ti) to acquire an image. FIG. 1 shows a temporal change in localization of NF-κB after LPS stimulation in the two-dimensional co-culture. In a condition in which R-7050 was not treated, the nuclear translocation of NF-κB was observed in only some cells at 0.5 hours and 1 hours after the LPS treatment, and the nuclear translocation of NF-κB was observed in many cells at 3 hours and 6 hours after the LPS treatment.

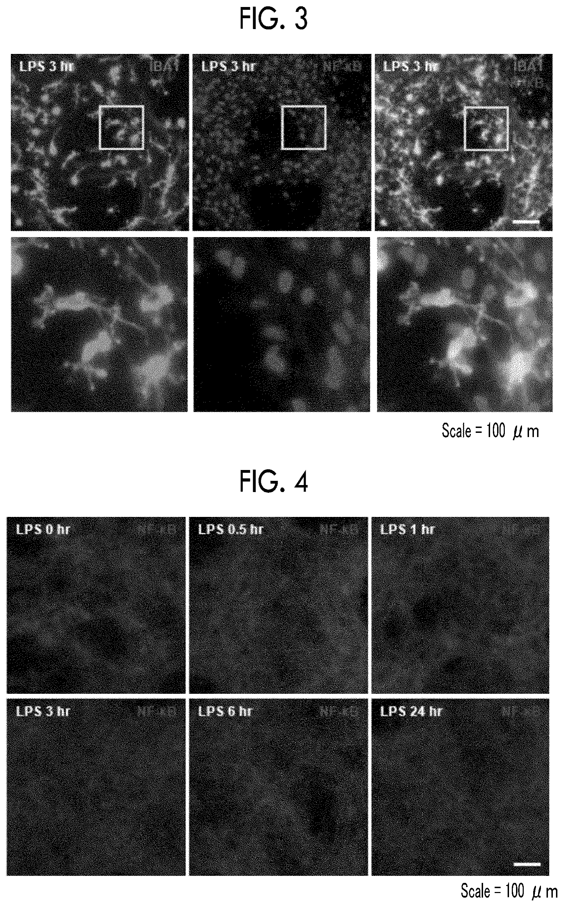

FIGS. 2 and 3 show a result of staining NF-κB and IBA1, which is a marker of microglia. NF-κB localized in the nucleus 0.5 hours after the LPS stimulation was co-localized with the microglia marker IBA1. On the other hand, at 3 hours after the LPS stimulation, NF-κB was localized in the nucleus in cells other than the IBA1-positive cells. That is, it was confirmed that, at 0.5 hours after the LPS stimulation, the microglia was activated and the nuclear translocation of NF-κB occurred, and then other cells were also activated and the nuclear translocation of NF-κB occurred. FIG. 4 shows a temporal change in localization of NF-κB after LPS stimulation in the co-culture of nerve and astrocytes. The nuclear translocation of NF-κB was not observed at any time. That is, from these results, it was confirmed that the activation of the microglia activated other cells and that the intercellular interaction occurred.

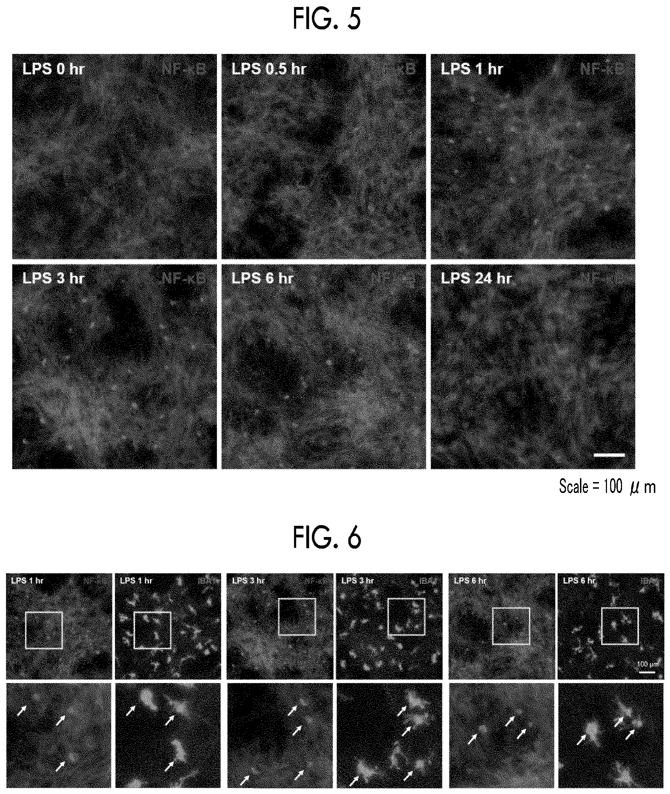

On the other hand, as shown in FIG. 5, in a condition in which R-7050 was treated, the nuclear translocation of NF-κB was confirmed in only some cells at 1, 3, and 6 hours after the LPS stimulation. In addition, as shown in FIG. 6, the nuclear signals of NF-κB at 1, 3, and 6 hours after the LPS stimulation were all co-localized with IBA1, which is a microglia marker, and it was confirmed that the nuclear translocation of NF-κB occurred in the microglia. From these results, it was shown that the activation of the microglia was suppressed from affecting other cells (intercellular interaction) by treating with the TNFR1 inhibitor. In addition, it was shown that the intercellular interaction between the microglia and other cells was mediated by TNF-α.

### <4> Localization evaluation of STAT 1

The fixed cells were washed with PBS(-), and blocking and permeabilization were performed by treating with a solution (1% BSA/0.2% Triton X-100) in which BSA (Sigma-Aldrich, A4161) and Triton (registered trademark) X-100 (BioVision, 2104-100) were diluted to 1% and 0.2% with PBS(-) for 30 minutes. Thereafter, as a primary antibody reaction, the cells were treated with an anti-IBA1 antibody (FUJIFILM Wako Pure Chemical Corporation, 011-27991) diluted 800-fold with 1% BSA/0.2% Triton X-100 and an anti-STAT1 antibody (Cell Signaling, 14994S) diluted 400-fold, and allowed to stand at 4°C overnight.

The next day, the cells were washed with PBS(-), then as a secondary antibody reaction, treated with Donkey anti-goat Alexa Fluor (registered trademark) 488 (Thermo Fisher Scientific, A11055) diluted 1000-fold, Donkey anti-rabbit Alexa Fluor (registered trademark) 594 (Thermo Fisher Scientific, A32754) diluted 1000-fold, and Hoechst (registered trademark) 33342 (Dojindo, 346-07951) diluted 1000-fold as a secondary antibody reaction, and allowed to stand at room temperature for 60 minutes. After washing with PBS(-), the cells were imaged with a fluorescence microscope (Nikon, ECLIPSE Ti) to acquire an image.

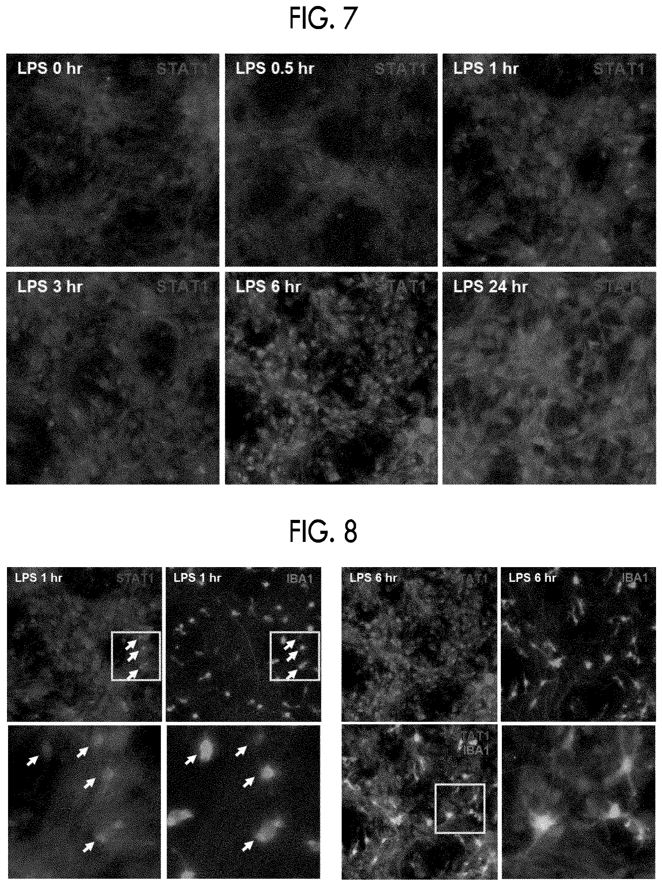

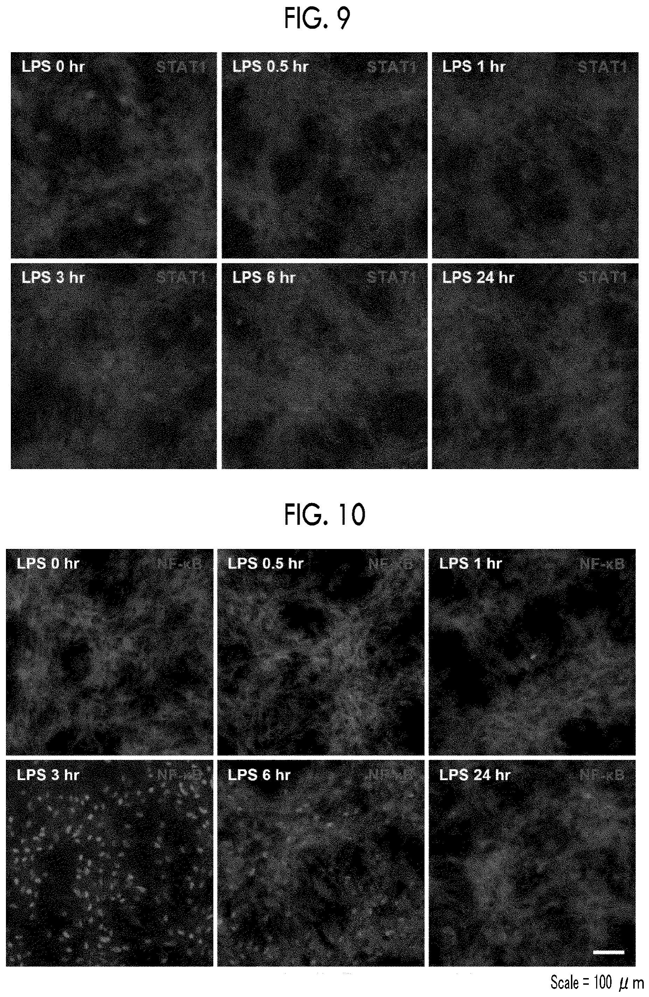

The localization evaluation of STAT1 was performed by the method described in <4> of Test Example 1. FIG. 7 shows a temporal change in localization of STAT1 after LPS stimulation in the three-cell-type co-culture system. STAT1 was localized in the nucleus in some cells at 1 hour and 3 hours after the LPS stimulation, but STAT1 was localized in the nucleus in many cells 6 at hours after the stimulation. FIG. 8 shows a result of staining STAT1 and IBA1, which is a marker of microglia. STAT1 localized in the nucleus at 1 hour after the LPS stimulation was co-localized with the microglia marker IBA1. On the other hand, at 6 hours after the LPS stimulation, STAT1 was localized in the nucleus in cells other than the IBA1-positive cells. That is, it was confirmed that, at 1 hour and 3 hours after the LPS stimulation, only the microglia was activated and the nuclear translocation of STAT1 occurred, and then other cells were also activated and the nuclear translocation of STAT1 occurred. FIG. 9 shows a temporal change in localization of STAT1 after LPS stimulation in the co-culture system of nerve and astrocytes. As a result, the nuclear translocation of STAT1 was not observed at any time. That is, from these results, it was confirmed that the activation of the microglia activated other cells and that the intercellular interaction occurred.

Test Example 2: Evaluation of intercellular interaction by immunostaining using three-dimensional three-cell-type co-culture system

### <1> Production of three-dimensional co-culture

Human iPS cell-derived astrocyte progenitor cells (XCell Science, XCS-AP-001-1V) were seeded in a flask coated with Matrigel basement membrane matrix (Corning, 354234) and cultured for 5 days (37°C, 5% CO₂) using a differentiation-inducing culture medium to differentiate into astrocytes.

**[Table 1]**

| Differentiation-inducing culture medium | | |
|---|---|---|
| Reagent name | Manufacturer/Model number | Final concentration |
| N2 Supplement with Transferrin (Apo) (×100) | FUJIFILM Wako Pure Chemical Corporation, 141-09041 | ×1 |
| GlutaMAX (trademark) | Thermo Fisher Scientific, 35050-061 | 1% |
| MEM Non-Essential Amino Acid | Thermo Fisher Scientific, 11140-050 | 1% |
| Penicillin-Streptomycin Solution (×100) | FUJIFILM Wako Pure Chemical Corporation, 168-23191 | ×0.5 |
| Heparin Sodium | FUJIFILM Wako Pure Chemical Corporation, 085-00134 | 0.5 U/mL |
| Animal-Free Recombinant Human CNTF | PeproTech, AF-450-13 | 10 ng/mL |
| Animal-Free Recombinant Human BMP-4 | PeproTech, AF-120-05ET | 10 ng/mL |

The differentiated astrocytes were peeled by TrypLE (trademark) Select (Thermo Fisher Scientific, 12563-029) and centrifuged at 600 × g at room temperature for 5 minutes. After centrifugation, the supernatant was removed, the cells were suspended in 1 mL of the co-culture medium, and the number of cells was counted.

The neurons produced by forcibly expressing the Ngn2 genes from the iPS cells was thawed in a warm bath at 37°C. After thawing, the cells were added to the co-culture medium and centrifuged at 600 × g at room temperature for 5 minutes. After centrifugation, the supernatant was removed, the cells were suspended in 1 mL of the co-culture medium, and the number of cells was counted.

The iPS cell-derived microglia (iCell (registered trademark) Microglia, FCDI, C1110) were thawed in a warm bath at 37°C. After thawing, the cells were added to the co-culture medium and centrifuged at 600 × g at room temperature for 5 minutes. After centrifugation, the supernatant was removed, the cells were suspended in 1 mL of the co-culture medium, and the number of cells was counted.

The astrocytes, the neurons, and the microglia were mixed at a ratio of 10:6:3, and seeded into a 96-well plate (Prime Surface (registered trademark) plate 96U, Sumitomo Bakelite, MS-9096U) at 1.9 × 10⁴ cells/well. The cells were cultured under the conditions of 37°C and 5% CO₂, and half of the culture medium was replaced three times a week.

**[Table 2]**

| Co-culture medium | | |
|---|---|---|
| Reagent name | Manufacturer/Model number | Final concentration |
| N2 Supplement with Transferrin (Apo) (×100) | FUJIFILM Wako Pure Chemical Corporation, 141-09041 | ×1 |
| GlutaMAX (trademark) | Thermo Fisher Scientific, 35050-061 | 1% |
| MEM Non-Essential Amino Acid | Thermo Fisher Scientific, 11140-050 | 1% |
| Penicillin-Streptomycin Solution (×100) | FUJIFILM Wako Pure Chemical Corporation, 168-23191 | ×0.5 |
| Animal-Free Recombinant Human CNTF | PeproTech, AF-450-13 | 10 ng/mL |
| Animal-Free Recombinant Human BMP-4 | PeproTech, AF-120-05ET | 10 ng/mL |
| 8-Br-cAMP | Sigma Aldrich, B7880 | 1 µmol/L |
| Animal-Free Recombinant Human/Murine/Rat BDNF | Peprotech, AF-450-02 | 20 ng/mL |
| Recombinant Human GDNF | Peprotech, 450-10 | 20 ng/mL |
| L(+)-Ascorbic Acid | FUJIFILM Wako Pure Chemical Corporation, 012-04802 | 20 nmol/L |
| Laminin | Sigma Aldrich, L2020 | 1 µg/mL |
| B-27 (registered trademark) Supplement (50X), serum free | Thermo Fisher Scientific, 17504044 | ×0.5 |

### <2> Intercellular interaction during neuroinflammation

The three-dimensional co-culture that had been cultured for 3 weeks was treated with Lipopolysaccharide (LPS) from E. coli 0127 (FUJIFILM Wako Pure Chemical Corporation, 124-05151) such that the final concentration was 100 ng/mL, and fixed by treating, for 30 minutes, with a 3% paraformaldehyde-phosphate buffer solution (FUJIFILM Wako Pure Chemical Corporation, 161-20141) diluted with PBS(-) (FUJIFILM Wako Pure Chemical Corporation, 166-23555) at 0, 0.5, 1, 3, 6, and 24 hours after the treatment. After washing with PBS (-), the co-culture was treated with Triton (registered trademark) X-100 (BioVision, 2104-100) diluted to 0.4% with PBS (-) for 15 minutes to perform permeation treatment. After the permeation treatment, the co-culture was treated with a BSA solution (Sigma-Aldrich, A416) prepared at a concentration of 4% with PBS (-) for 30 minutes to perform blocking. Thereafter, as a primary antibody reaction, the cells were treated with an anti-IBA1 antibody (FUJIFILM Wako Pure Chemical Corporation, 011-27991) diluted 1000-fold and an anti-NF-κB p65 antibody (Cell Signaling, 8242) diluted 800-fold, and allowed to stand at 4°C overnight.

The next day, the cells were washed with PBS(-), then as a secondary antibody reaction, treated with Donkey anti-goat Alexa Fluor (registered trademark) 488 (Thermo Fisher Scientific, A11055) diluted 1000-fold, Donkey anti-rabbit Alexa Fluor (registered trademark) 594 (Thermo Fisher Scientific, A32754) diluted 1000-fold, and Hoechst (registered trademark) 33342 solution (Dojindo, H342) diluted 1000-fold as a secondary antibody reaction, and allowed to stand at room temperature for 60 minutes. After washing with PB S(-), imaging was performed with a confocal quantitative image cytometer CQ1 (Yokogawa Electric Corporation, Cell Voyager (registered trademark) CQ1) to acquire images.

### Test Example 3: Evaluation of amount of microglia required to induce intercellular interaction

The amount of cells required for the activated microglia to affect other cells was examined. Two-dimensional three-cell-type co-cultures of astrocytes, neurons, and microglia in which the proportion of the microglia was about 11%, 1.1%, and 0.11% were produced by the same method as in <1> of Test Example 1. In addition, the cells were subjected to LPS stimulation and fixed by the same method as in <1> of Test Example 1. Furthermore, the localization of NF-κB was evaluated by the same method as in <1> of Test Example 1.

FIG. 10 shows a temporal change in localization of NF-κB in the three-cell-type co-culture in which the proportion of the microglia is about 0.11%. As a result, the nuclear translocation of NF-κB was confirmed in only some cells at 1 hour after the LPS treatment, and the nuclear translocation of NF-κB was confirmed in many cells at 3 hours and 6 hours after the LPS treatment.

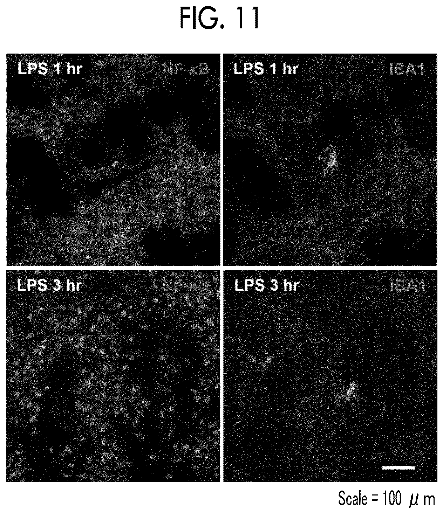

FIG. 11 shows a result of staining NF-κB and IBA1, which is a marker of microglia. The signal of NF-κB localized in the nucleus at 1 hour after the LPS stimulation was co-localized with the microglia marker IBA1. On the other hand, at 3 hours after the LPS stimulation, NF-κB was localized in the nucleus in cells other than the IBA1-positive cells. That is, it was confirmed that, even in the three-cell-type co-culture in which the proportion of the microglia was about 0.11%, the microglia was first activated after the LPS stimulation, and then other cells were activated, and the intercellular interaction occurred.

### Test Example 4: Evaluation of intercellular interaction by calcium imaging

### <1> Production of two-cell-type co-culture of astrocytes and neurons, and three-cell-type co-culture of astrocytes, neurons, and microglia

Matrigel basement membrane matrix (Corning, 356234) diluted 120-fold with DMEM/F12 (Life technologies, 11320-033) was added to a 96-well plate at 65 µL/well and allowed to stand at 4°C. After 24 hours, the culture medium was replaced with a progenitor cell culture medium and used.

Astrocyte precursor cells (XCell Science, XCS-AP-001-1V) were seeded in a 96-well plate at a density of 6 × 10⁴ cells/well, cultured under the conditions of 37°C and 5% CO₂, after culture medium was replaced with the differentiation induction culture medium, further cultured for 5 days to differentiate into astrocytes.

The neurons were produced by forcibly expressing the Ngn2 genes from the iPS cells and the cells that had been frozen and stored were used. The cells that had been frozen and stored were thawed in a warm bath at 37°C, and after thawing, the cells were added to the co-culture medium and centrifuged at 600 × g at room temperature for 5 minutes. After centrifugation, the supernatant was removed, the cells were suspended in 1 mL of the co-culture medium, and the number of cells was counted.

As the microglia, iPS cell-derived microglia (iCell (registered trademark) Microglia, FCDI, C1110) were used. The cells that had been frozen and stored were thawed in a warm bath at 37°C, and after thawing, the cells were added to the co-culture medium and centrifuged at 600 × g at room temperature for 5 minutes. After centrifugation, the supernatant was removed, the cells were suspended in 1 mL of the co-culture medium, and the number of cells was counted.

In the two-cell-type co-culture, the neurons were seeded on the astrocytes at 3 × 10⁴ cells/well. In the three-cell-type co-culture, the neurons and the microglia were seeded on the astrocytes, each at 3 × 10⁴ cells/well. The cells were cultured under the conditions of 37°C and 5% CO₂, and half of the culture medium was replaced once a week.

### <2> Activation of microglia or astrocytes, and evaluation of neural activity by calcium imaging

After co-culturing for 4 weeks, the microglia and the astrocytes were stimulated by adding LPS (FUJIFILM Wako Pure Chemical Corporation, 124-05151) at 100 ng/mL or TNF-α (Cell signaling technology, 8902SC) at 7.5 ng/mL.

After 3 hours of the stimulation with TNF-α, the cells were fixed by the method described in <1> of Test Example 1, and the localization of NF-κB was evaluated by the same method as in <1> of Test Example 1.

In addition, at 24 hours after the stimulation with LPS or TNF-α, the neural activity was evaluated by calcium imaging. The working solution and the recording solution were adjusted by adding the reagents shown in Tables 3 and 4 to HBSS(-).

**[Table 3]**

| Working solution | | |
|---|---|---|
| Reagent name | Manufacturer/Model number | Concentration |
| Calcium chloride dihydrate | FUJIFILM Wako Pure Chemical Corporation, 038-19735 | 2.5 mmol/L |
| Calbryte (trademark) 520AM | AAT Bioquest, 20651 | 4 µmol/L |
| Pluronic F-127 (20% solution in DMSO) | Thermo fisher scientific, P3000MP | 0.04% |
| HEPES buffer Solution | FUJIFILM Wako Pure Chemical Corporation, 345-00681 | 10 mmol/L |

**[Table 4]**

| Recording solution | | |
|---|---|---|
| Reagent name | Manufacturer/Model number | Concentration |
| Calcium chloride dihydrate | FUJIFILM Wako Pure Chemical Corporation, 038-19735 | 2.5 mmol/L |
| HEPES buffer solution | FUJIFILM Wako Pure Chemical Corporation, 345-06681 | 10 mmol/L |
| PBS (-) | FUJIFILM Wako Pure Chemical Corporation, 166-23555 | 50% |

50 µL of the culture medium was removed from a well containing 100 µL of the culture medium, and 50 µL/well of a working solution warmed at 37°C was added. After incubating for 1 hour under conditions of 37°C and 5% CO₂, the solution was replaced with a warmed (37°C) recording solution, and the fluorescence was measured for 10 minutes with FDSS (registered trademark)/µcell (Hamamatsu Photonics K.K., C13299). The neural activity was evaluated by quantifying the number of peaks of calcium oscillation using Wave Checker Software (Hamamatsu Photonics K.K.).

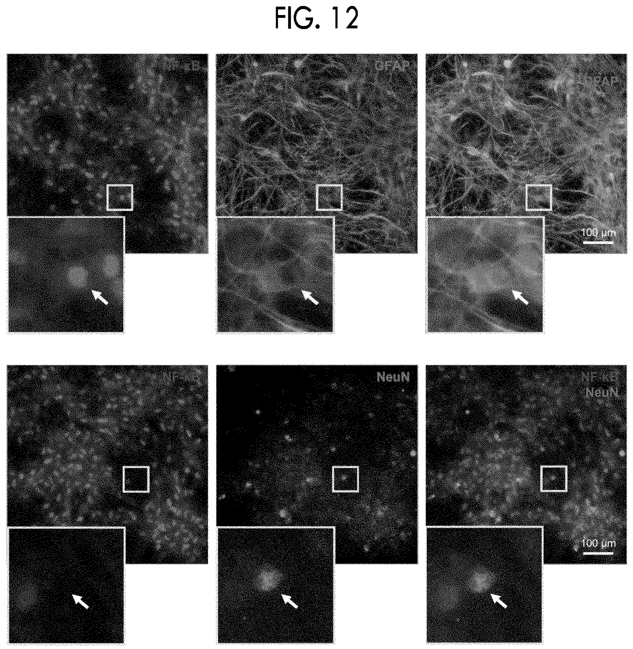

FIG. 12 shows a result of staining NF-κB, GFAP, and Neun in a case where the two-dimensional two-cell-type co-culture of neurons and astrocytes was stimulated with TNF-α. It was confirmed that in a case where the two-dimensional two-cell-type co-culture of neurons and astrocytes was stimulated with TNF-α, the nuclear translocation of NF-κB occurred in the GFAP-positive cells, which are the markers of the astrocytes, at 3 hours after the stimulation, and the astrocytes were activated. On the other hand, the nuclear translocation of NF-κB was not confirmed in the Neun-positive cells, which are the markers of the neurons. That is, it was shown that TNF-α stimulated only the astrocytes in the co-culture.

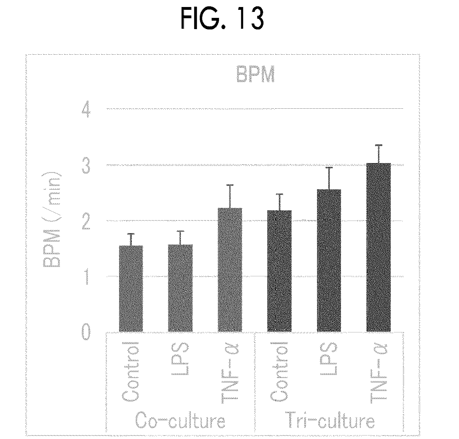

FIG. 13 shows the results of the calcium imaging in the two-dimensional two-cell-type co-culture of neurons and astrocytes and the two-dimensional three-cell-type co-culture of neurons, astrocytes, and microglia. As a result of stimulating the astrocytes with TNF-α and performing calcium imaging 24 hours later, increase of the BPM (the number of peaks of calcium oscillation per minute), that is, enhancement of the neural activity was confirmed in both the two-cell-type and three-cell-type co-cultures as compared with the control. Since the increase in the BPM was also confirmed in the two-cell-type co-culture, it was confirmed that the astrocytes stimulated by TNF-α enhanced the neural activity, that is, the intercellular interaction occurred between the astrocytes and the neurons. In addition, there was no change in the BPM in a case where the two-cell-type co-culture was stimulated with LPS, but in a case where the three-cell-type co-culture was stimulated with LPS, the increase in the BPM was confirmed as compared with the control. In the three-cell-type co-culture, it was shown in Test Example 1 that the microglia was activated by the LPS stimulation to release TNF-α, and then the TNF-α activated the astrocytes. From these results, it was shown that, in the three-cell-type co-culture stimulated with LPS, first, the microglia was activated as the first cells, then the astrocytes as the second cells were activated via TNF-α, and thereby the activity of the neurons, which are the third cells, was increased, and it was confirmed that the intercellular interaction occurred in the three cells.

## Claims

1. A method of evaluating intercellular interactions in neuroinflammation, the method comprising:
a step of producing a co-culture containing at least two cells selected from the group consisting of human-derived astrocytes, human-derived neurons, human-derived microglia, and human-derived oligodendrocytes;
a step of applying an inflammatory stimulation to first cells contained in the co-culture;
a step of detecting at least one selected from the group consisting of an inflammatory response marker in the cells contained in the co-culture, neural activity of the cells, and cell morphology; and
a step of evaluating, over time, a change in at least one selected from the group consisting of the inflammatory response marker, the neural activity, and the cell morphology in the first cells and second cells different from the first cells and contained in the co-culture.

2. The method according to claim 1,
wherein the astrocytes, the neurons, the microglia, and the oligodendrocytes are induced to differentiate from human-derived pluripotent stem cells.

3. The method according to claim 2,
wherein the human-derived pluripotent stem cells are human iPS cells.

4. The method according to any one of claims 1 to 3,
wherein the first cells are human-derived microglia, human-derived astrocytes, or human-derived neurons.

5. The method according to any one of claims 1 to 3,
wherein, in a case where the first cells are human-derived astrocytes, the second cells are at least one selected from the group consisting of human-derived neurons and human-derived microglia.

6. The method according to any one of claims 1 to 3,
wherein, in a case where the first cells are human-derived neurons, the second cells are at least one selected from the group consisting of human-derived astrocytes and human-derived microglia.

7. The method according to any one of claims 1 to 3,
wherein, in a case where the first cells are human-derived microglia, the second cells are at least one selected from the group consisting of human-derived astrocytes and human-derived neurons.

8. The method according to any one of claims 1 to 3,
wherein the co-culture is a two-dimensional culture or a three-dimensional culture.

9. The method according to any one of claims 1 to 3,
wherein the inflammatory stimulation is a chemical stimulation.

10. The method according to claim 9,
wherein the chemical stimulation is a chemical stimulation with at least one substance selected from the group consisting of lipopolysaccharide, rotenone, amyloid-β protein, tau protein, α-synuclein, and TDP-43.

11. The method according to any one of claims 1 to 3,
wherein the inflammatory stimulation is a stimulation by adding cells different from the cells in the co-culture or adding a culture supernatant obtained by culturing the cells different from the cells in the co-culture.

12. The method according to any one of claims 1 to 3,
wherein the inflammatory stimulation is induced by aging of the first cells, a genetic mutation in the first cells, or gene introduction into the first cells in the co-culture.

13. The method according to claim 12,
wherein a protein produced by the genetic mutation in the first cells in the co-culture or the gene introduction into the first cells in the co-culture is at least one selected from the group consisting of amyloid-β protein, tau protein, α-synuclein, and TDP-43.

14. The method according to any one of claims 1 to 3,
wherein the inflammatory stimulation that stimulates the first cells is induced by a substance secreted from cells different from the first cells in the co-culture.

15. The method according to any one of claims 1 to 3,
wherein the inflammatory response marker is at least one selected from the group consisting of NF-κB, TNF-α, and STAT1.

16. The method according to any one of claims 1 to 3,
wherein the step of detecting the inflammatory response marker in the cells contained in the co-culture is a step of visualizing the inflammatory response marker protein by an immunostaining method.

17. The method according to any one of claims 1 to 3,
wherein the step of detecting the inflammatory response marker in the cells contained in the co-culture is a step of visualizing an mRNA expression level of the inflammatory response marker gene by a fluorescent in situ hybridization method.

18. The method according to any one of claims 1 to 3,
wherein the neural activity is at least one selected from the group consisting of intracellular calcium flux and action potential of the cells.

19. The method according to any one of claims 1 to 3,
wherein the step of detecting the neural activity in the cells contained in the co-culture is a step of visualizing intracellular calcium flux.

20. The method according to claim 19,
wherein the step of visualizing the intracellular calcium flux is calcium imaging.

21. The method according to any one of claims 1 to 3,
wherein the step of detecting the neural activity in the cells contained in the co-culture is a step of measuring action potential of the cells.
